# EUROPEAN PATENT SPECIFICATION

(11) **EP 4 126 132 B1**
(45) Date of publication and mention of the grant of the patent: **11.03.2026**
(21) Application number: 21715655.3
(22) Date of filing: 01.04.2021
(51) Int. Cl.: A61M 5/32

(54) **INJECTION DEVICE WITH NEEDLE OBSCURING ELEMENT**
INJEKTIONSVORRICHTUNG MIT NADELVERDECKUNGSELEMENT
DISPOSITIF D'INJECTION AVEC ÉLÉMENT MASQUANT L'AIGUILLE

(30) Priority: 03.04.2020 EP 20315113
(43) Date of publication of application: 08.02.2023
(73) Proprietor: SANOFI, 75017 Paris (FR)
(72) Inventor: DASBACH, Uwe, 65926 Frankfurt am Main (DE); SCHEINERT, Kai, 65926 Frankfurt am Main (DE); AUERNHAMMER, Daniel, 65926 Frankfurt am Main (DE); SCHAUDERNA, Florian, 65926 Frankfurt am Main (DE); KEMP, Thomas Mark, Melbourn, Herts Cambridgeshire SG8 6DP (GB); SCHULLER, Tim, Melbourn, Herts Cambridgeshire SG8 6DP (GB); WILSON, Robbie, Melbourn, Herts Cambridgeshire SG8 6DP (GB); NOBLE, Michael, Melbourn, Herts Cambridgeshire SG8 6DP (GB); MCGINLEY, Ryan Anthony, Melbourn, Herts Cambridgeshire SG8 6DP (GB)
(74) Representative: Colonna, Matthew Oliver
(86) International application number: PCT/EP2021/058755
(87) International publication number: WO 2021/198486

(56) References cited:
- EP-A1- 2 468 328
- DE-U1- 202004 017 971
- US-A- 5 147 303
- US-A- 5 419 773

## Description

### FIELD OF INVENTION

The present invention relates to an injection device having a needle obscuring element for obscuring a needle.

### BACKGROUND

Injection devices, such as auto-injectors, are known in the art for dispensing a medicament to the injection site of a patient. Such injection devices often comprise a body and a cap, a needle syringe located in the body and the cap removably attached to the body to shield the needle of the needle syringe. To dispense the medicament, the cap is first removed from the body to expose the needle. The needle is then inserted into the body of the patient at the injection site to dispense the medicament.

Some patients may be anxious regarding the injection process. For example, some patients may have a fear of needles, making the injection process off-putting for them. There is a desire to reduce patient anxiety surrounding an injection process. EP2468328 A1 discloses an auto-injector comprising movable spring arms. US 5147303A discloses a syringe held within a sheath having an opening sealed by a pivoted spring biased disc. US 5419773 A discloses a syringe with automatically actionable protective needle cover.

### SUMMARY

It is an object of the present invention to provide an injection device having a needle obscuring element. According to the present invention, there is provided an injection device according to claim 1.

According to the present invention, there is provided an injection device comprising: a needle; a needle sleeve surrounding the needle, the needle sleeve having an aperture and being arranged such that the needle extends through the aperture during an injection process; and a needle obscuring element movable between a first position and a second position relative to the needle sleeve, wherein the needle obscuring element is coupled to the needle sleeve when in the first position and when in the second position, and wherein the needle obscuring element is arranged to cover a larger portion of the aperture when in the second position than when in the first position.

The needle obscuring element may be biased towards the second position. This can ensure the needle is automatically obscured by default, reducing patient anxiety.

The needle obscuring element may be arranged to move from the first position to the second position in response to removal of a cap from the needle sleeve of the injection device. This provides a simple mechanism for ensuring the needle remains obscured prior to an injection.

The injection device may further comprise a housing, wherein the needle is fixed relative to the housing, and wherein the needle sleeve is configured to move into the housing. This may provide a simple mechanism for allowing the needle to be exposed through the aperture of the needle sleeve during an injection.

The injection device may further comprise a housing, wherein the needle sleeve is fixed relative to the housing, and wherein the needle is configured to move relative to the needle sleeve. This may provide a simple mechanism for allowing the needle to be exposed through the aperture of the needle sleeve during an injection.

The needle obscuring element may comprise at least one flap arranged within the needle sleeve, wherein the at least one flap is pivotally coupled to the needle sleeve such that the at least one flap can pivot between the first position and the second position. This can provide an implementation of the needle obscuring element that is simple to manufacture and which is easy to operate.

The at least one flap may be configured to pivot between the first position and the second position around an axis of rotation substantially parallel to the aperture. This can provide a compact implementation of the needle obscuring element.

The at least one flap may be coupled to the needle sleeve by a living hinge, wherein the at least one flap is biased towards the second position by the living hinge. This can provide a simple implementation of the needle obscuring element with minimal parts.

The needle obscuring element may comprise an iris diaphragm or a resilient diaphragm. Use of an iris diaphragm or a resilient diaphragm can provide a compact, simple implementation of the needle obscuring element.

The needle obscuring element may be arranged to have an opening when in the second position, through which the needle may extend during the injection process. This can allow the needle obscuring means to remain in position before, during and after an injection process, reducing patient anxiety.

The needle obscuring element may be integrally formed with the needle sleeve. This provides a straightforward implementation of the needle obscuring means that is simple to manufacture. For example, the needle obscuring element and needle sleeve may be integrally formed using an injection moulding process.

The needle obscuring element may be configured to lock in the second position in response to moving from the first position to the second position. This may improve the safety of the injection device, by limiting access to the needle inside the needle sleeve. This may also reduce patient anxiety by ensuring the needle obscuring element remains in the second position, obscuring the needle.

The injection device may further comprise a cap having a needle shield, wherein the needle shield is at least partially located within the needle sleeve, and wherein the needle obscuring element is held in the first position by the needle shield when the needle shield is at least partially located within the needle sleeve. This may provide a simple manner of allowing the needle obscuring means to move from the first position to the second position.

The injection device may be an auto-injector, a pen-injector, a safety syringe, or a large-volume injection device.

The injection device may further comprise a container containing a medicament.

The needle obscuring element can reduce the visibility of the needle to the user, thereby reducing patient anxiety. The needle obscuring element may also increase the safety of the injection device by restricting access to the needle by a user. The needle obscuring element may also maintain the sterility of the injection device by helping prevent the ingress of foreign bodies such as dirt into the needle shield and so near the needle.

### BRIEF DESCRIPTION OF THE DRAWINGS

Embodiments of the invention will now be described, by way of example only, with reference to the accompanying drawings, in which:
FIG. 1A is a schematic side view of an injection device that embodies the invention, and a removable cap;
FIG. 1B is a schematic side view of the injection device of FIG. 1A, with the cap removed from the housing;
FIG. 2 is an enlarged cross-sectional side view of the distal end of an injection device according to an embodiment of the invention, with the needle obscuring element in a first position;
FIG. 3 is an enlarged cross-sectional side view of the distal end of the injection device of FIG. 2, with the needle obscuring element in a second position;
FIG. 4 is an enlarged, three-dimensional view of the distal end of injection device of FIG. 2;
FIG. 5 is an enlarged, three-dimensional view of the distal end of injection device of FIG. 3;
FIG. 6 is a three-dimensional partial view of a flap shown in FIG. 4, coupled to the needle sleeve;
FIG. 7 is a view of the injection device of FIG.2 when viewed along the longitudinal axis of the injection device, from the distal end towards the proximal end;
FIG. 8 is a view of the injection device of FIG.3 when viewed along the longitudinal axis of the injection device, from the distal end towards the proximal end;
FIG. 9 is an enlarged cross-sectional side view of the distal end of an injection device according to another embodiment of the invention, with the needle obscuring element in a first position;
FIG. 10 is an enlarged cross-sectional side view of the distal end of the injection device of FIG. 9, with the needle obscuring element in a second position;
FIG. 11 is a view of the injection device of FIG.9 when viewed along the longitudinal axis of the injection device, from the distal end towards the proximal end;
FIG. 12 is a view of the injection device of FIG.10 when viewed along the longitudinal axis of the injection device, from the distal end towards the proximal end;
FIG. 13 is an enlarged cross-sectional side view of the distal end of an injection device according to yet another embodiment of the invention, with the needle obscuring element in a first position;
FIG. 14 is an enlarged cross-sectional side view of the distal end of the injection device of FIG. 13, with the needle obscuring element in a second position;
FIG. 15 is a view of the injection device of FIG.13 when viewed along the longitudinal axis of the injection device, from the distal end towards the proximal end;
FIG. 16 is a view of the injection device of FIG.14 when viewed along the longitudinal axis of the injection device, from the distal end towards the proximal end;
FIG. 17 is an enlarged cross-sectional side view of the distal end of an injection device according to yet another embodiment of the invention, with the needle obscuring element in a first position;
FIG. 18 is an enlarged cross-sectional side view of the distal end of the injection device of FIG. 17, with the needle obscuring element in a second position.

### DETAILED DESCRIPTION

A drug delivery device, as described herein, may be configured to inject a medicament into a patient. For example, delivery could be sub-cutaneous, intra-muscular, or intravenous. Such a device could be operated by a patient or care-giver, such as a nurse or physician, and can include various types of safety syringe, pen-injector, or auto-injector. The device can include a cartridge-based system that requires piercing a sealed ampule before use. Volumes of medicament delivered with these various devices can range from about 0.5 ml to about 3 ml. Another device can include a large volume device ("LVD") or patch pump, configured to adhere to a patient's skin for a period of time (e.g., about 5, 15, 30, 60, or 120 minutes) to deliver a "large" volume of medicament (typically about 2 ml to about 10 ml). Yet another device may comprise a pre-filled syringe within a housing of the device. The syringe may be fixed within the housing or may be moveable within the housing, for example from a retracted position to an operation extended position.

In combination with a specific medicament, the presently described devices may also be customized in order to operate within required specifications. For example, the device may be customized to inject a medicament within a certain time period (e.g., about 3 to about 20 seconds for auto-injectors, and about 10 minutes to about 60 minutes for an LVD). Other specifications can include a low or minimal level of discomfort, or to certain conditions related to human factors, shelf-life, expiry, biocompatibility, environmental considerations, etc. Such variations can arise due to various factors, such as, for example, a drug ranging in viscosity from about 3 cP to about 50 cP. Consequently, a drug delivery device will often include a hollow needle ranging from about 25 to about 31 Gauge in size. Common sizes are 17 and 29 Gauge.

The delivery devices described herein can also include one or more automated functions. For example, one or more of combining the needle and cartridge, needle insertion, medicament injection, and needle retraction can be automated. Energy for one or more automation steps can be provided by one or more energy sources. Energy sources can include, for example, mechanical, pneumatic, chemical, or electrical energy. For example, mechanical energy sources can include springs, levers, elastomers, or other mechanical mechanisms to store or release energy. One or more energy sources can be combined into a single device. Devices can further include gears, valves, or other mechanisms to convert energy into movement of one or more components of a device.

The one or more automated functions of an auto-injector may each be activated via an activation mechanism. Such an activation mechanism can include an actuator, for example, one or more of a button, a lever, a needle sleeve, or other activation component. Activation of an automated function may be a one-step or multi-step process. That is, a user may need to activate one or more activation components in order to cause the automated function. For example, in a one-step process, a user may depress a needle sleeve against their body in order to cause injection of a medicament. Other devices may require a multi-step activation of an automated function. For example, a user may be required to depress a button and retract a needle shield in order to cause injection.

In addition, activation of one automated function may activate one or more subsequent automated functions, thereby forming an activation sequence. For example, activation of a first automated function may activate at least two of combining the needle and cartridge, needle insertion, medicament injection, and needle retraction. Some devices may also require a specific sequence of steps to cause the one or more automated functions to occur. Other devices may operate with a sequence of independent steps.

Some delivery devices can include one or more functions of a safety syringe, pen-injector, or auto-injector. For example, a delivery device could include a mechanical energy source configured to automatically inject a medicament (as typically found in an auto-injector) and a dose setting mechanism (as typically found in a pen-injector).

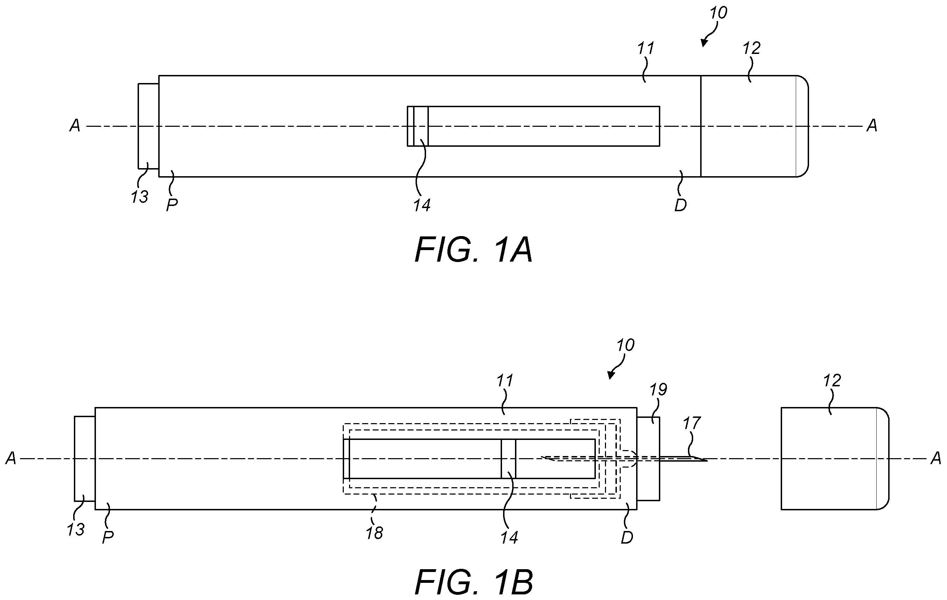

According to some embodiments of the present disclosure, an exemplary drug delivery device 10, also known as an injection device, is shown in FIGS. 1A and 1B. Device 10, as described above, is configured to inject a medicament into a patient's body. Device 10 includes a housing 11 which typically contains a cartridge or pre-filled syringe that defines a reservoir containing the medicament to be injected, and the components required to facilitate one or more steps of the delivery process.

The device 10 can also include a cap 12 that can be detachably mounted to the housing 11. Typically, a user must remove cap 12 from housing 11 before device 10 can be operated.

As shown, housing 11 is substantially cylindrical and has a substantially constant diameter along the longitudinal axis A-A. The housing 11 has a distal region D and a proximal region P. The term "distal" refers to a location that is relatively closer to a site of injection, and the term "proximal" refers to a location that is relatively further away from the injection site.

Device 10 can also include a needle sleeve 19 coupled to housing 11 to permit movement of sleeve 19 relative to housing 11. For example, sleeve 19 can move in a longitudinal direction parallel to longitudinal axis A-A. Specifically, movement of sleeve 19 in a proximal direction can permit a needle 17 to extend from distal region D of housing 11.

Insertion of needle 17 can occur via several mechanisms. For example, needle 17 may be fixedly located relative to housing 11 and initially be located within an extended needle sleeve 19. Proximal movement of sleeve 19 by placing a distal end of sleeve 19 against a patient's body and moving housing 11 in a distal direction will uncover the distal end of needle 17. Such relative movement allows the distal end of needle 17 to extend into the patient's body. Such insertion is termed "manual" insertion as needle 17 is manually inserted via the patient's manual movement of housing 11 relative to sleeve 19.

Another form of insertion is "automated", whereby needle 17 moves relative to housing 11. Such insertion can be triggered by movement of sleeve 19 or by another form of activation, such as, for example, a button 13. As shown in FIGS. 1A and 1B, button 13 is located at a proximal end of housing 11. However, in other embodiments, button 13 could be located on a side of housing 11.

Other manual or automated features can include drug injection or needle retraction, or both. Injection is the process by which a bung or piston 14 is moved from a proximal location to a more distal location within the reservoir of the medicament container 18 in order to force a medicament from the container 18 through needle 17. In some embodiments, a drive spring (not shown) is under compression before device 10 is activated. A proximal end of the drive spring can be fixed within proximal region P of housing 11, and a distal end of the drive spring can be configured to apply a compressive force to a proximal surface of piston 14. Following activation, at least part of the energy stored in the drive spring can be applied to the proximal surface of piston 14. This compressive force can act on piston 14 to move it in a distal direction. Such distal movement acts to compress the liquid medicament within the container 18, forcing it out of needle 17.

Following injection, needle 17 can be retracted within sleeve 19 or housing 11. Retraction can occur when sleeve 19 moves distally as a user removes device 10 from a patient's body. This can occur as needle 17 remains fixedly located relative to housing 11. Once a distal end of sleeve 19 has moved past a distal end of needle 17, and needle 17 is covered, sleeve 19 can be locked. Such locking can include locking any proximal movement of sleeve 19 relative to housing 11.

Another form of needle retraction can occur if needle 17 is moved relative to housing 11. Such movement can occur if the cartridge 18 within housing 11 is moved in a proximal direction relative to housing 11. This proximal movement can be achieved by using a retraction spring (not shown), located in distal region D. A compressed retraction spring, when activated, can supply sufficient force to the cartridge 18 to move it in a proximal direction. Following sufficient retraction, any relative movement between needle 17 and housing 11 can be locked with a locking mechanism. In addition, button 13 or other components of device 10 can be locked as required.

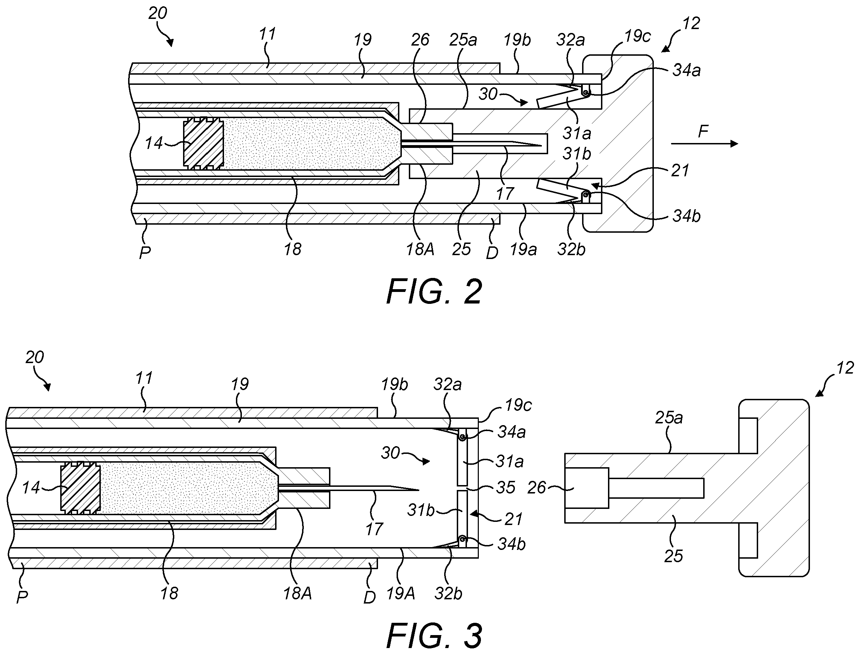

Referring now to Figure 2, part of an injection device 20 according to an example embodiment of the invention is shown. The injection device 20 is in the form of an auto-injector that has similar features to the device 10 described above in relation to Figures 1A and 1B, with like features retaining the same reference numerals. However, it should be understood that the invention may apply to other types of injection device 20.

A medicament container 18 is provided within the housing 11 of the injection device 20. The container 18 contains liquid medicament which is sealed by a piston 14 located within a reservoir of the container 18. The container 18 may be a cartridge or a syringe, for example. In an initial state, the piston 14 is positioned at a position closest to the proximal end P of the housing 11. A driving mechanism is provided at the proximal end P of the housing 11 which is arranged to push the piston 14 towards the distal end D of the housing 11 once it is in an active state and when it is actuated. A hollow injection needle 17 is coupled to the container 18 so that the needle 17 is in fluid communication with the reservoir of the container 18. The needle 17 may not initially be in fluid communication with the reservoir of the container 18, but may instead be moved into fluid communication. For example the needle 17 may be actuated to pierce a septum of the container 18. When the needle 17 is in fluid communication with the reservoir of the container 18, medicament is displaced through the needle 17 by the piston 14 being moved towards the distal end D of the housing 11.

The cylindrical needle sleeve 19 extends from the distal end D of the housing 11 in a direction parallel to the longitudinal axis of the injection device 20. The needle sleeve 19 circumferentially surrounds the needle 17 and has an aperture 21 at a distal end formed by the inner surface 19a of the needle sleeve 19. The needle 17 protrudes through the aperture 21 during an injection process.

In an initial state, before an injection takes place, the needle sleeve 19 extends along the longitudinal axis of the injection device 20 beyond the tip of the needle 17 thereby shielding the needle 17. The needle sleeve 19 shields the exposed end of the needle 17 and therefore prevents both unintentional damage of the needle 17 during handling and access of a user to the needle 17 for avoiding stick injuries.

During an injection state, however, the tip of the needle 17 extends beyond the needle sleeve 19 so that the needle 17 is able to pierce the patient. This is achieved by the needle sleeve 19 being able to move relative to the needle 17 in a direction parallel to the longitudinal axis. In some examples, the needle sleeve 19 is able to move relative to the housing 11 along the longitudinal axis A-A, while the needle 17 remains in a fixed longitudinal position relative to the housing 11. Before an injection, the needle sleeve 19 extends beyond the tip of the needle 17. To perform an injection, the needle sleeve 19 is moved relative to the housing 11 along the longitudinal axis A-A in a proximal direction. For example, the needle sleeve 19 may be translated into the housing 11. Movement of the needle sleeve 19 relative to the housing 11 could be in response to a user placing the distal end of the needle sleeve 19 on an injection site and applying a force in the distal direction, thereby causing the housing 11 and needle 17 to move towards the injection site while the needle sleeve 19 translates into the housing 11.

In other examples, the needle sleeve 19 is fixed relative to the housing 11, while the needle 17 is able to move relative to the housing 11 and needle sleeve 19 along the longitudinal axis A-A. Before an injection, the needle sleeve 19 extends beyond the tip of the needle 17. To perform an injection, the needle 17 is moved relative to the needle sleeve 19 and housing 11 in a distal direction along the longitudinal axis A-A, thereby causing at least the tip of the needle 17 to protrude beyond the needle sleeve 19.

A removable cap 12 is coupled to a distal end of the injection device 20. The cap 12 is inserted into the needle sleeve 19 via the aperture 21. Like the needle sleeve 19, the cap 12 can prevent both unintentional damage of the needle 17 during handling of the injection device 20 and access of a user to the needle 17 for avoiding stick injuries.

The removable cap 12 may comprise a needle shield 25. The needle shield 25 has a recess 26 that is configured to receive a portion 18A of the container 18 that is housed in the housing 11 of the injection device 20. The friction between the needle shield 25 and the end portion 18A of the container 18 is sufficient to hold the needle shield 25 and cap 12 in place, covering the needle 17. Alternatively, or in addition, the cap 12 may be coupled to one or more other parts of the injection device 20, such as the needle sleeve 19 or the housing 11. Furthermore, the cap 12 may be coupled to the injection device 20 by a friction fit, by an engagement mechanism such as cooperating ridges, grooves, pips or bumps on the cap 12 and injection device 20, by an adhesive, or by another suitable coupling means.

The needle shield 25 provides additional protection to the needle 17 by further shielding the needle 17, for example to prevent physical damage to the needle 17 during handling of the injection device 20, or to maintain sterility of the needle 17.

Before an injection takes place, the cap 12 is removed from the injection device 20 by pulling the cap 12 in the direction F along the longitudinal axis of the injection device 20, thereby removing the needle shield 25 from around the needle 17 and exposing the needle 17 to the inside of the needle sleeve 19. The needle 17 may now be visible to the user, when viewing the inside of the needle sleeve 19 through the aperture 21.

Figure 2 shows the injection device 20 also having a needle obscuring element 30. The needle obscuring element 30 is configured to obscure (i.e. conceal or hide) the needle 17 to a user. When reference is made to obscuring the needle 17, this means that the needle 17 is made less visible to a user, or it is made more difficult for a user to view the needle 17. Obscuring the needle 17 does not necessarily mean the needle 17 is entirely hidden from the user, but this may be the case. The needle obscuring element 30 makes the needle 17 less visible to a user than if the needle obscuring element 30 were not present. In particular, the needle obscuring element 30 obscures the visibility of the needle 17 when viewed through the aperture 21 from the distal end D of the injection device 20. At least part of the needle obscuring element 30 may be opaque or translucent, to reduce the visibility of the needle 17.

The needle obscuring element 30 is arranged at a distal end D of the injection device 20. Figure 2 shows the needle obscuring element 30 located at a distal end of the needle sleeve 19, proximate the aperture 21. The needle obscuring element 30 may be coupled to the needle sleeve 19, such as an inner surface 19a of the needle sleeve 19.

The needle obscuring element 30 is movable between a first position and a second position, relative to the needle sleeve 19. The needle obscuring element 30 is arranged to cover a larger portion (i.e. area) of the aperture 21 when in the second position than when in the first position. Therefore the visibility of the needle 17 through the aperture 21 is more obscured when the needle obscuring element 30 is in the second position than in the first position. A biasing element may bias the needle obscuring element 30 towards the second position. The needle 17 may therefore be more obscured by default, with little or no input from the user.

Figure 2 shows the needle obscuring element 30 in a first position. Figure 3 shows the same injection device 30 as Figure 2, however Figure 3 shows the needle obscuring element 30 in a second position. The needle obscuring element 30 may be coupled to the needle sleeve 19 when in the first position, and remain coupled to the needle sleeve 19 when in the second position.

The needle obscuring element 30 can be envisaged in a number of manners, as will be discussed below.

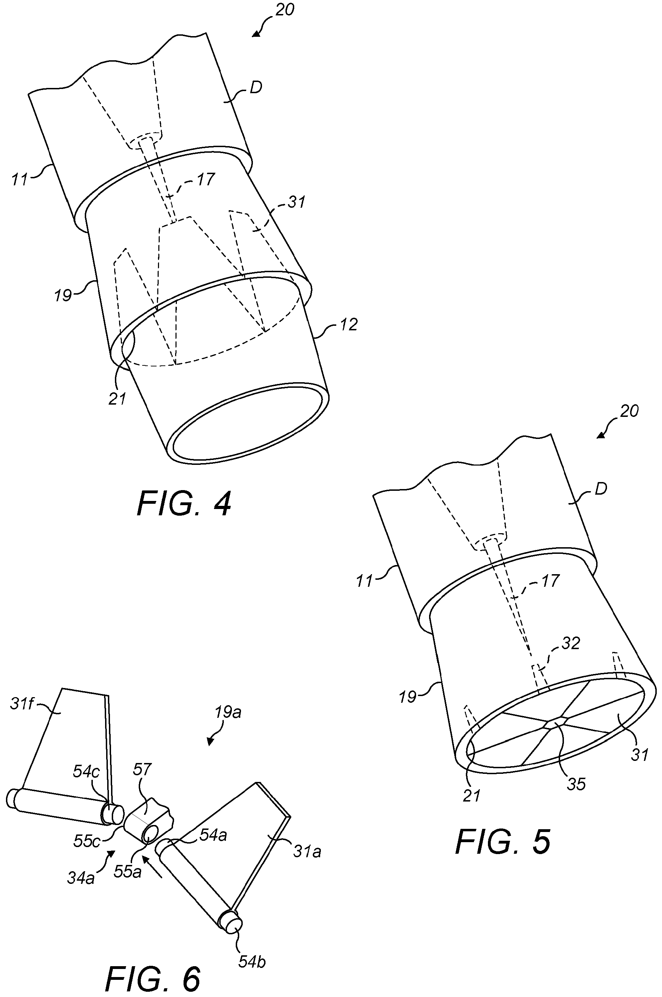

Figure 2 shows the needle obscuring element 30 comprising a plurality of flaps 31. While only a first flap 31a and a second flap 31b are visible in the cross-section of Figure 2, there are actually six flaps 31 present in the needle obscuring element 30, as shown more clearly in Figures 4, 5, 7 and 8. However, it should be understood that the number of flaps 31 is not limited to six, and could be greater than or less than six. For example, one flap 31 or two or more flaps 31 may be used.

Each flap 31a,b is coupled to the needle sleeve 19. Figure 2 shows each flap 31a,b coupled to the inner surface 19a of the needle sleeve 19. However the flaps 31 could be coupled to another part of the needle sleeve 19, for example an outer surface 19b of the needle sleeve 19 or an end surface 19c of the needle sleeve 19. The flaps 31 are arranged circumferentially around the aperture 21, proximate the distal end of the needle sleeve 19. The flaps 31 are located between the needle 17 and the distal end of the injection device 20, when the injection device 20 is in the initial state before an injection.

The flaps 31 are coupled to the needle sleeve 19 by any suitable coupling means, for example by a hinge, a ball joint, or the like. Figure 2 shows the first flap 31a coupled to the inner surface 19a of the needle shield 19 by a first hinge 34a and the second flap 31b coupled to the inner surface 19a of the needle shield 19 by a second hinge 34b. The flaps 31 are coupled so that they are able to move between the first position and second position. Figure 6, discussed later, shows an example of how the flaps 31 are coupled to the needle sleeve 19.

Each flap 31a,b has a respective resilient member 32a,b acting as a biasing element to bias the respective flap 31a,b towards the second position. Figure 2 shows each resilient member 32a,b comprising a protrusion formed from a resilient material and extending from a respective flap 31a,b. The resilient member 32a,b may be integrally formed with the respective flap 31a,b.

Each resilient member 32a,b contacts the inner surface 19a of the needle sleeve 19. When the flaps 31 are in the first position, the resilient members 32 are in a stressed (e.g. compressed or stretched) state, exerting a force on the inner surface 19a of the needle sleeve 19 in an attempt to push the flaps 31 into the second position.

In some examples each resilient member 32 is coupled to the needle sleeve 19 rather than to the flaps 31, but makes contact with a respective flap 31 when the flaps 31 are in the first position. In this case, when the flaps 31 are in the first position, the resilient members 32 are in a stressed state, and exert a force on the respective flaps 31 in an attempt to push the flaps 31 into the second position.

Any type of resilient member 32 suitable for biasing the flaps 31 from the first position to the second position could be used. For example, the resilient member 32 could comprise one or more springs or the like. Figures 2 and 3 show each flap 31 having a respective resilient member 32, however the present disclosure is not limited to such an example. For example, one resilient member 32 may act as the biasing element for more than one flap 31. In some cases, one resilient member 32 may act as the biasing element for all flaps 31.

In some examples the resilient member 32 may comprise a living hinge. One side of the living hinge may be coupled to the needle sleeve 19 and the other side coupled to a flap 31. In this case, the flaps 31 and resilient members 32 may be integrally formed with the needle sleeve 19, for example in an injection moulding process. The living hinge may act as both the coupling means for coupling the flap 31 to the needle sleeve 19 as well as the biasing element for biasing the flap 31 from the first position towards the second position.

Figure 2 shows the flaps 31 in the first position relative to the needle sleeve 19. In this case each flap 31a,b extends in a direction substantially parallel to the longitudinal axis of the injection device 20, perpendicular to the aperture 21.

Before an injection takes place, the cap 12 is removed from the injection device 20 by pulling the cap 12 in the direction 'F' along the longitudinal axis of the injection device 20. As the cap 12 is removed from the injection device 20, the needle shield 25 uncovers the needle 17.

Figure 3 shows the injection device 20 of Figure 2 after the cap 12 has been removed. The needle 17 is now exposed within the needle sleeve 19. However, the flaps 31 have now moved from their first position to their second position, obscuring the needle 17 when viewed through the aperture 21 from the distal end of the injection device 20. The flaps 31 have each pivoted from the first position to the second position, about respective axes that are substantially perpendicular to the longitudinal axis of the injection device 20.

The needle obscuring element 30 has been moved from the first position to the second position in response to the removal of the needle cap 12 from the needle sleeve 19. In Figure 2 the flaps 31 were in the first position but were biased towards the second position by the resilient members 32. The flaps 31 were held against an outer surface 25a of the needle shield 25, with the needle shield 25 holding the flaps 31 in the first position. However upon removal of the cap 12 and needle shield 25, as shown in Figure 3, the flaps 31 are no longer held in the first position by the needle shield 25 and so they automatically move into the second position due to the force exerted by the resilient members 32.

An opening 35 is still provided in the needle obscuring element 30 when in the second position. This allows the needle 17 to pass through the needle obscuring element 30 during an injection. However, the size of the opening 35 is smaller than the size of the aperture 21, meaning that a user is less able to view the needle 17 when the needle obscuring element 30 is in the second position than in the first position.

While Figure 2 shows the flaps 31 being held in the first position by the needle shield 25 of the cap 12, in other examples the flaps 31 may be held in the first position by a different part of the cap 12 other than the needle shield 25.

In some examples the needle obscuring element 30 comprises a locking member which is configured to lock the needle obscuring element 30 in the second position, after the needle obscuring element 30 has moved from the first position to the second position. Locking the needle obscuring element 30 in the second position may improve the safety of the device by restricting access to the needle 17 by a user.

While the injection device 20 of Figures 2 and 3 has been described as having a biasing element (e.g. resilient members 32) for biasing the needle obscuring element 30 towards the second position, in other examples such a biasing element may not be present. For example, the needle obscuring element 30 may not be biased towards the second position, however the needle obscuring element 30 is still movable between the first position and second position, for example by an action of the user.

Figure 4 is a three-dimensional view of part of the injection device 20 of Figure 2, when viewed from distal end of the injection device 20. As can be seen in Figure 4, the cap 12 is inserted into the needle sleeve 19. The needle sleeve 19 and cap 12 have been rendered transparent in Figure 4 for illustration purposes, in order to better show the arrangement of the flaps 31 within the needle sleeve 19.

The cap 12 is holding the flaps 31 in the first position so that they extend substantially along a direction parallel to the longitudinal axis of the injection device 20. As discussed previously, six flaps 31a-f are shown in Figures 4 and 5, however more flaps 31 or fewer flaps 31 than this may be present.

Figure 5 shows the injection device 20 of Figure 4 after the cap 12 has been removed. The flaps 31 are now in the second position in which they cover a substantial portion of the aperture 21 of the needle sleeve 19, as described previously with reference to Figure 3. The flaps 31 remain coupled to the needle sleeve 19 between the first position and the second position, however the flaps 31 now cover a larger portion of the aperture 21 when in the second position compared to the first position, as can be seen when comparing Figure 5 to Figure 4. The needle 17 is therefore obscured by the flaps 31 in the second position.

The flaps 31 still form an opening 35 when in the second position, through which the needle 17 may protrude during an injection. However, it can be seen from Figure 5 that visibility of the needle 17 is reduced due to the presence of the flaps 31 in the second position.

Figure 6 shows an example of how the flaps 31 can be coupled to the needle sleeve 19, for example as shown in the injection device 20 of Figures 2-5.

Figure 6 shows each flap 31 having a first protrusion 54a and a second protrusion 54b arranged at opposing side surfaces of the flap 31a and extending along the same axis of rotation. Coupling members 57a,b,c protrude from the needle sleeve 19, each coupling member 57 spaced apart circumferentially around the inner surface 19a of the needle sleeve 19. Each flap 31 is located between a respective pair of coupling members 57. The first protrusion 54a and second protrusion 54b of each flap 31 are received in a respective first receiving portion 55a and second receiving portion 55b of the coupling members 57, so that the flaps 31 are rotatably coupled to the needle sleeve 19 and can pivot between the first position and the second position. In some examples, one or more of the protrusions 54 may be formed on a coupling member 57, while one or more of the corresponding receiving portions 55 may be formed on a flap 31.

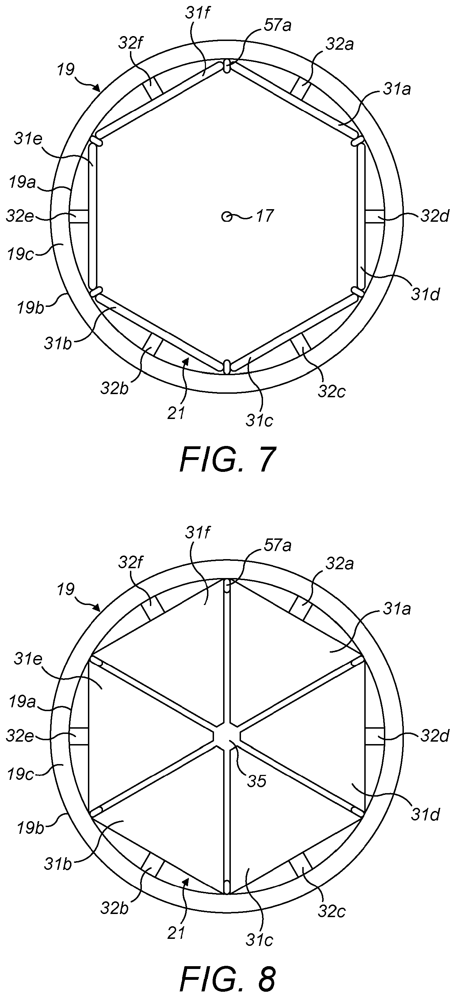

Figure 7 shows the injection device 20 of Figures 2 and 4 when viewed end-on, along the longitudinal axis from the distal end towards the proximal end of the injection device 20. For illustration purposes, the cap 12 is not shown in Figure 7. Nevertheless, Figure 7 shows the flaps 31 as they would appear in the first position, for example if the cap 12 were coupled to the injection device 20, or immediately after removal of the cap 12 before the flaps 31 have moved to the second position.

As can be seen in Figure 7, the six flaps 31a-f are in their first position and so cover only a relatively small portion of the aperture 21 when compared to the second position shown in Figure 8. As a result, the needle 17 may be visible to a user when viewed from the distal end of the injection device 20 through the aperture. The resilient members 32a-f are in a stressed state between the inner surface 19a of the needle sleeve 19 and their respective flaps 31a-f, thereby biasing the flaps 31a-f towards the second positon. However, it should be noted that in some examples the resilient members 32 are not present, and so the flaps 31 are not biased towards the second position.

Figure 8 shows the same view of the injection device 20 of Figure 7, however the flaps 31a-f are now in the second position. This may occur after removal of the cap 12 from the injection device 20, for example after a non-zero period of time after removal, once the flaps 31 have been able to move from the first position to the second position. The flaps 31a-f may have been caused to move into the second position by the resilient members 32a-f. It can be seen a greater portion of the aperture 21 has been covered by the flaps 31 when in the second position of Figure 8 compared to the first position of Figure 7.

The flaps 31 still form an opening 35 when in the second position, through which the needle 17 is able protrude during an injection. However, it can be seen from Figure 7 that the visibility of the needle 17 is reduced due to the presence of the flaps 31a-f in the second position. The presence of the flaps 31a-f in the second position may reduce the amount of light that enters the needle sleeve 19 via the aperture 21, making the needle 17 more difficult to see.

While the needle obscuring element 30 has been discussed with reference to Figures 2 to 8 as comprising a plurality of flaps 31, the present disclosure is not limited to such an example.

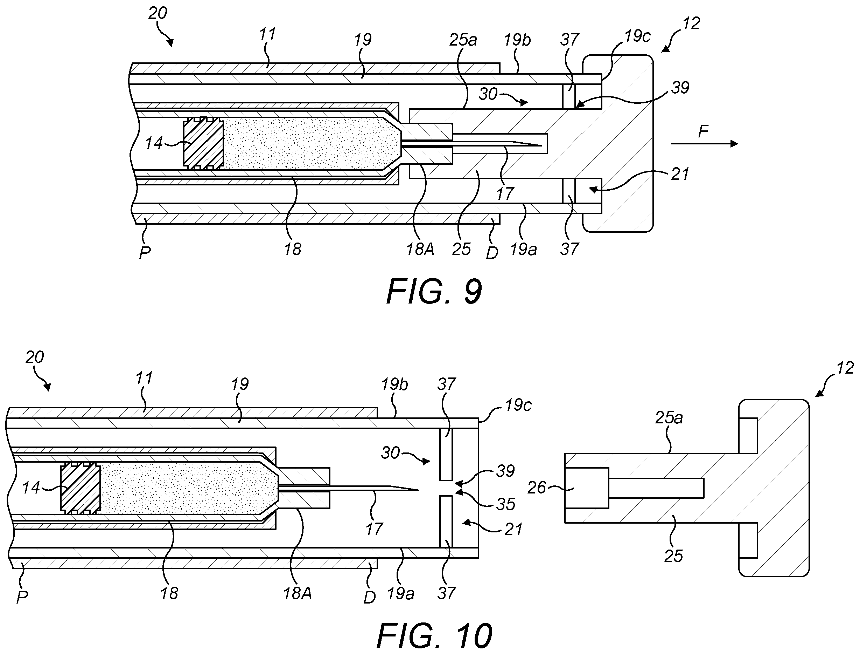

Figures 9 to 12 show another embodiment in accordance with aspects of the present disclosure.

Figures 9 and 10 show a similar injection device 20 to Figures 2 and 3, however the needle obscuring element 30 instead comprises an iris diaphragm 37.

The iris diaphragm 37 is coupled to the needle sleeve 19 so that it extends across the aperture 21.

The iris diaphragm 37 comprises leaves 38, also known as blades, arranged in a circular arrangement to form a diaphragm aperture 39 in the middle. Each leaf 38 is able to pivot about an axis extending parallel to the longitudinal axis of the injection device 20. By pivoting the leaves 38, the size of the diaphragm aperture 39 may be changed. A smaller diaphragm aperture 39 may allow less of the needle 17 to be seen through the aperture 21 compared to a larger diaphragm aperture 39.

Figures 9 and 10 show the iris diaphragm 37 in a first position and a second positon respectively. In the first position shown in Figure 9, the diaphragm aperture 39 is larger than in the second position shown in Figure 10. As such, the needle 17 is only partially obscured by the iris diaphragm 37 when viewed from the distal end of the injection device 20 through the aperture 21.

In the second position shown in Figure 10, the diaphragm aperture 39 is smaller than in the second position shown in Figure 9. As such, the needle 17 is obscured by the iris diaphragm 37 when viewed from the distal end of the injection device 20 through the aperture 21, by a greater amount than when in the first position. It can be seen in Figure 10 that the diaphragm aperture 39 acts as an opening 35when the iris diaphragm 37 is in the second position, so that the needle 17 may protrude through the iris diaphragm 37 during an injection.

The iris diaphragm 37 may be biased towards the second position by a biasing element in a similar manner as discussed for other embodiments. This may be achieved, for example by a resilient member 32 coupled to one or more leaves 38 of the iris diaphragm 37. When a cap 12 is coupled to the injection device 20, the iris diaphragm 37 may be held in the first position in a similar manner as described in relation to Figure 2. That is, the leaves 38 may be urged against the outer surface 25a of the needle shield 25 by the resilient members 32. Upon removal of the cap 12, the leaves 38 are no longer held in place by the needle shield 25 and so the iris diaphragm 37 moves into the second position due to the forces exerted by the resilient members 32.

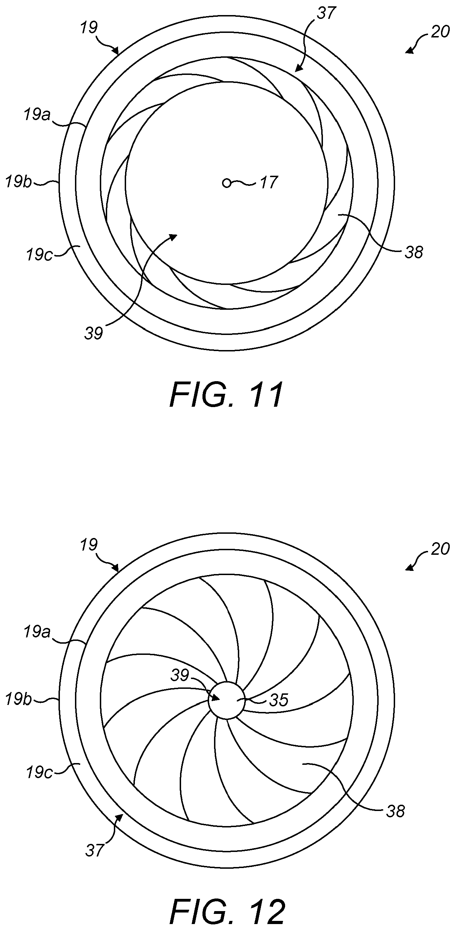

Figure 11 shows the injection device 20 of Figure 9 when viewed end-on, along the longitudinal axis from the distal end towards the proximal end of the injection device 20. For illustration purposes, the cap 12 is not shown in Figure 11. Nevertheless, Figure 11 shows the iris diaphragm 37 as it would appear in the first position, for example if the cap 12 were coupled to the injection device 20, or immediately after removal of the cap 12 before the iris diaphragm 37 has moved to the second position.

As can be seen in Figure 11, the leaves 38 of the iris diaphragm 37 are in their first position, forming a relatively large diaphragm aperture 39. As such, the iris diaphragm 37 covers only a relatively small portion of the aperture 21 of the needle sleeve 19 when compared to the iris diaphragm 37 in the second position shown in Figure 12. As a result, the needle 17 may be visible to a user when viewed from the distal end of the injection device 20 through the aperture. The iris diaphragm 37 may be biased towards the second positon by a biasing element. However, it should be noted that in some examples the biasing element is not present, and so the iris diaphragm 37 is not biased towards the second position. Instead, the iris diaphragm 37 may be moved between the first position and second position by a user.

Figure 12 shows the same view of the injection device 20 of Figure 11, however the iris diaphragm 37 is now in the second position. This may occur after removal of the cap 12 from the injection device 20, for example after a non-zero period of time after removal, once iris diaphragm 37 has been able to move from the first position to the second position. The iris diaphragm 37 may have been caused to move into the second position by a biasing element. The leaves 38 of the iris diaphragm 37 have moved to form a smaller diaphragm aperture 39 than when in the first position. As such, it can be seen that a greater portion of the aperture 21 is covered by the iris diaphragm 37 when in the second position of Figure 12 compared to the first position of Figure 11.

The diaphragm aperture 39 still acts an opening 35 when the iris diaphragm 37 is in the second position, through which the needle 17 is able protrude during an injection. However, it can be seen from Figure 12 that the visibility of the needle 17 would be reduced due to the presence of the iris diaphragm 37 in the second position. The presence of the iris diaphragm 37 in the second position may reduce the amount of light that enters the needle sleeve 19 via the aperture 21, making the needle 17 more difficult to see.

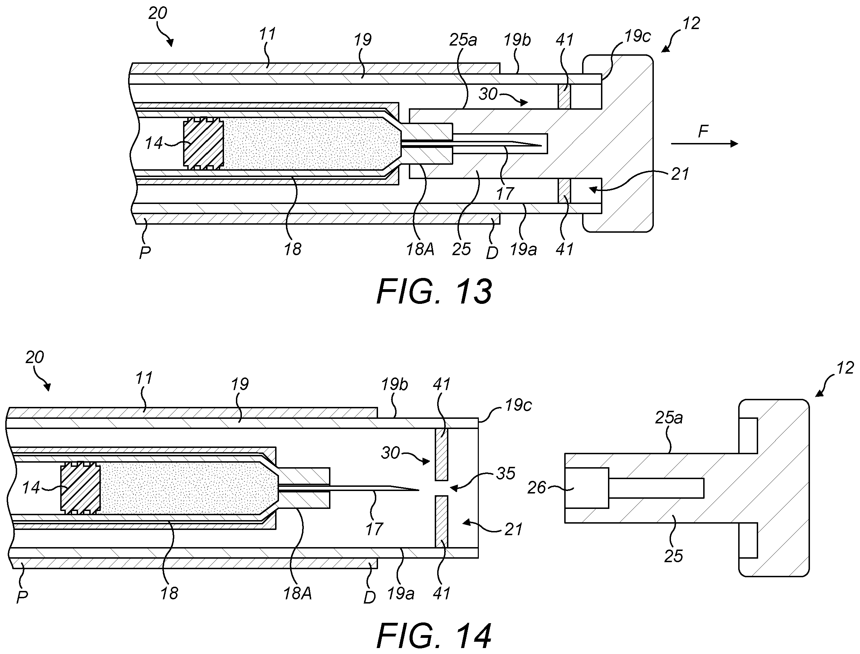

Figures 13 to 16 show another embodiment in accordance with aspects of the present disclosure. The injection device 20 is similar to the injection device of Figures 2-12, however in this embodiment the needle obscuring element 30 comprises a resilient diaphragm 41. The resilient diaphragm 41 is coupled to the needle sleeve 19 and extends across at least part of the aperture 21. The resilient diaphragm 41 is shown coupled to the inner surface 19a of the needle sleeve 19, but it may alternatively be coupled to an outer surface 19b or an end surface 19c of the needle sleeve 19. The resilient diaphragm 41 may be formed from an elastic or viscoelastic material. The resilient diaphragm 41 is a flexible piece of material that can change its shape.

Figure 13 shows the resilient diaphragm 41 in its first position, for example when the cap 12 is attached to the injection device 20. The resiliency of the resilient diaphragm 41 biases the resilient diaphragm 41 towards its second positon shown in Figure 14, however the presence of the cap 12 (i.e. needle shield 25) holds the resilient diaphragm 41 in the first position.

Figure 14 shows the resilient diaphragm 41 of Figure 13 when in the second position, for example some time after removal of the cap 12 from the injection device 20. The resiliency of the resilient member 41 has caused it to revert to the second position, expanding to increase its surface area and cover more of the aperture than when in the first position. The visibility of the needle 17 is therefore obscured more than when the resilient diaphragm 41 was in the first position. The resilient diaphragm 41 still has an opening 35 through which the needle 17 may protrude during an injection.

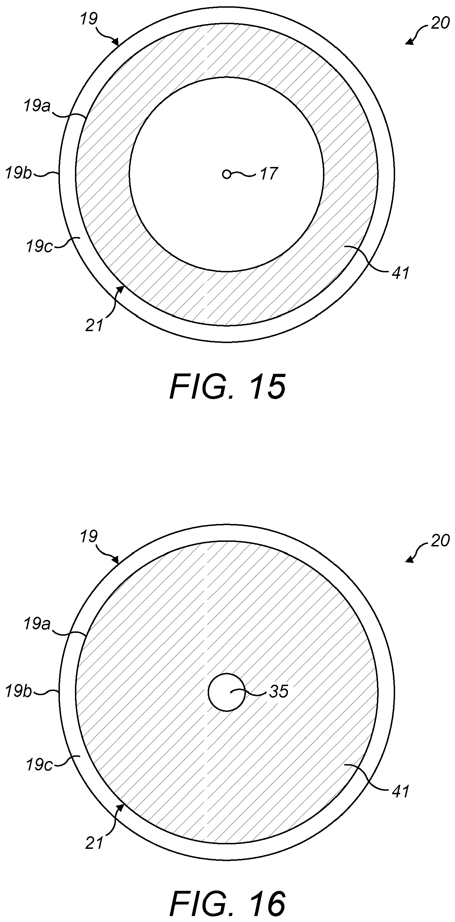

Figures 15 and 16 show the injection device 20 of Figures 13 and 14 respectively,, when viewed along the longitudinal axis of the injection device 20 from the distal end of the injection device 20 towards the proximal end of the injection device 20.

When the resilient diaphragm 41 is in the first position, as shown in Figure 15, it covers a relatively smaller area of the aperture 21 than compared to being in the second position, as shown in Figure 16. The visibility of the needle 17 is therefore obscured more when the resilient diaphragm 41 is in the second position than when in the first position. The resiliency of the material of the resilient diaphragm 41 biases the resilient diaphragm 41 towards the second position.

In the examples of Figures 13-16, the resilient diaphragm 41 is shown as a sheet of flexible material coupled to the needle sleeve 19 that stretches across the aperture 21.

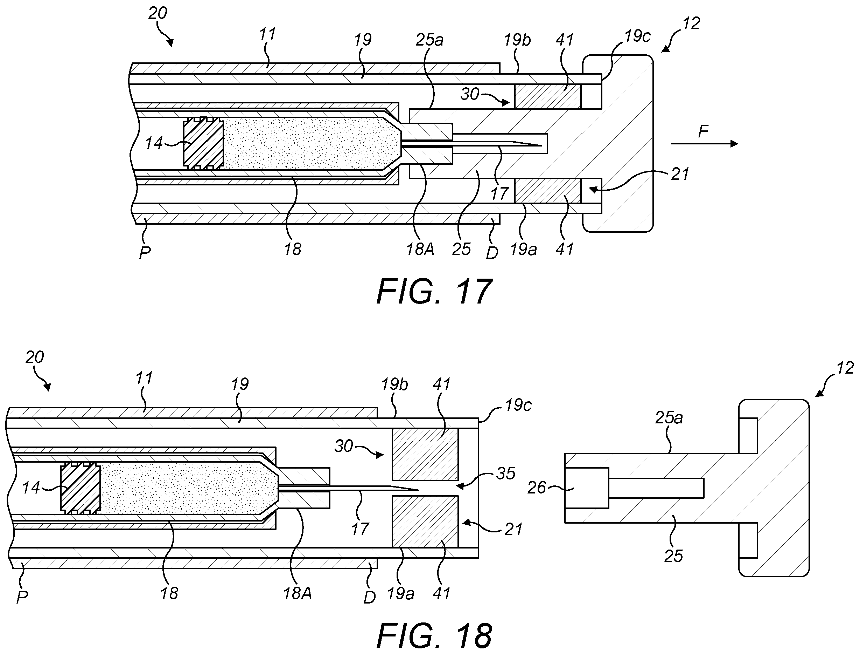

However, in other examples the resilient diaphragm 41 may have a more three-dimensional structure. For example, Figures 17 and 18 show a similar injection device 20 to Figures 13 and 14 respectively, however in Figures 17 and 18 the resilient diaphragm 41 comprises a tube of resilient material that extends a more substantial distance down the length of the needle sleeve 19. In such an example, the resilient diaphragm 41 may be formed from a tube of resilient foam such as polyurethane foam.

Figure 17 shows the resilient diaphragm 41 in the first position while Figure 18 shows the resilient diaphragm 41 in the second position. In the first position the tube of resilient foam is in a compressed state, with the resilient diaphragm 41 held in the compressed state by the needle shield 25 of the cap 12. Figure 18 shows the injection device 20 after the cap 12 has been removed. The needle shield 25 is no longer located in the needle sleeve 19. The tube of resilient foam moves from the compressed state to an expanded state, taking up a larger area inside the needle sleeve 19 and covering a larger portion of the aperture 21. The resilient diaphragm 41 moves from the first state to the second state due to the resilient nature of the resilient diaphragm 41.

The resilient diaphragm 41 has an opening 35 when in the second position as shown in Figure 18, which allows the needle 17 to extend through the opening during an injection process.

In each of the examples discussed herein, the needle obscuring element 30 has been described as forming an opening 35 when in the second position to allow the needle 17 to extend through the needle obscuring element 30 during an injection process. However, in some examples an opening 35 may not be present when the needle obscuring element 30 is in the second position. For example where the needle obscuring element 30 comprises a resilient diaphragm 41, the resilient diaphragm 41 may expand to the second position such that any opening 35 is completely sealed. This may further obscure the needle 17, as well as improve the safety of the injection device 20 and further reduce the possibility of ingress of foreign bodies into the needle sleeve 19. However in such a scenario, the needle obscuring element 30 is constructed such that the needle 17 is still able to extend through the needle obscuring element 30 during an injection process. This may be achieved by the needle obscuring element 30 being formed from a material that the needle 17 is able to pierce through during an injection process, or that the needle 17 is able to deform in order to pass through.

It will be appreciated that in the various embodiments described above, the needle obscuring element 30 is generally moveable between the first and second positions, and is moveable in a number of ways within the various embodiments described, for example by pivoting, flexing, resiliently deflecting, rotating and expanding. In some examples, the needle obscuring element 30 is movable from the first position to the second position. Additionally or alternatively, in some examples the needle obscuring element 30 is movable from the second position to the first position.

It will be appreciated that in the various embodiments described above, the needle obscuring element 30 can be biased towards the second position. However in some examples in accordance with the present invention, the needle obscuring element 30 may not biased towards the second position. For example, a user may manually move the needle obscuring element 30 from the first position to the second position.

It will be appreciated that in the various embodiments described above, the needle obscuring element 30 is generally held in the first position by a cap 12, and that removal of the cap 12 allows the needle obscuring element to move from the first position to the second position. However in some examples in accordance with the present invention, a cap 12 is not present. For example, the needle obscuring element 30 may be held in the first position by a different part. In yet other examples, the needle obscuring element 30 is not held in the first position, but is movable between the second position and first position.

In various embodiments described above, the needle obscuring element 30 has been said to comprise an iris diaphragm 37 or a resilient diaphragm 41. However, it will be appreciated that other suitable types of diaphragm, may be used as the needle obscuring element 30, as long as the diaphragm is able to move between a first position and second position, where the diaphragm covers a larger portion of the aperture 21 when in the second position compared to when in the first position. For example, the diaphragm may have a variable aperture.

The provision of a needle obscuring element 30 that is movable between a first position and a second position is advantageous in that it can allow for access to the needle 17 and inside of the needle sleeve 19 when in the first position, but obscure the visibility of the needle 17 when in the second position. For example, the first position may allow the cap 12, and in particular a needle shield 25, to be positioned within the needle sleeve 19, around the needle 17. However after the cap 12 is removed, the needle obscuring element 30 moves to the second position to hide the needle 17 from the user.

A needle obscuring element 30 that is movable between a first position and a second position may be advantageous where parts of the injection device 20 are to be assembled through the aperture 21 and needle shield 19. For example, one or more of the medicament container 18 or needle 17 may need to be inserted into the housing 11 of the injection device 20 via the aperture 21 and needle shield 19 prior to an injection. The needle obscuring element 30 may be in the first position to allow for loading of the medicament container 18 and/or needle 17 into the injection device 20 via the aperture 21 and needle shield 19, but may move to the second position after loading is complete.

The embodiments of injector devices described herein are configured to receive either a cartridge of medicament or a syringe pre-filled with a medicament. Herein, the term "medicament container" is intended to encompass both a cartridge of medicament and a pre-filled syringe.

The terms "drug" or "medicament" are used herein to describe one or more pharmaceutically active compounds. As described below, a drug or medicament can include at least one small or large molecule, or combinations thereof, in various types of formulations, for the treatment of one or more diseases. Exemplary pharmaceutically active compounds may include small molecules; polypeptides, peptides and proteins (e.g., hormones, growth factors, antibodies, antibody fragments, and enzymes); carbohydrates and polysaccharides; and nucleic acids, double or single stranded DNA (including naked and cDNA), RNA, antisense nucleic acids such as antisense DNA and RNA, small interfering RNA (siRNA), ribozymes, genes, and oligonucleotides. Nucleic acids may be incorporated into molecular delivery systems such as vectors, plasmids, or liposomes. Mixtures of one or more of these drugs are also contemplated.

The term "drug delivery device" shall encompass any type of device or system configured to dispense a drug into a human or animal body. Without limitation, a drug delivery device may be an injector device (e.g., syringe, pen injector, auto injector, large-volume device, pump, perfusion system, or other device configured for intraocular, subcutaneous, intramuscular, or intravascular delivery), skin patch (e.g., osmotic, chemical, micro-needle), inhaler (e.g., nasal or pulmonary), implantable (e.g., coated stent, capsule), or feeding systems for the gastro-intestinal tract. The presently described drugs may be particularly useful with injector devices that include a needle, e.g., a small gauge needle.

The drug or medicament may be contained in a primary package or "drug container" adapted for use with a drug delivery device. The drug container may be, e.g., a cartridge, syringe, reservoir, or other vessel configured to provide a suitable chamber for storage (e.g., short- or long-term storage) of one or more pharmaceutically active compounds. For example, in some instances, the chamber may be designed to store a drug for at least one day (e.g., 1 to at least 30 days). In some instances, the chamber may be designed to store a drug for about 1 month to about 2 years. Storage may occur at room temperature (e.g., about 20°C), or refrigerated temperatures (e.g., from about - 4°C to about 4°C). In some instances, the drug container may be or may include a dual-chamber cartridge configured to store two or more components of a drug formulation (e.g., a drug and a diluent, or two different types of drugs) separately, one in each chamber. In such instances, the two chambers of the dual-chamber cartridge may be configured to allow mixing between the two or more components of the drug or medicament prior to and/or during dispensing into the human or animal body. For example, the two chambers may be configured such that they are in fluid communication with each other (e.g., by way of a conduit between the two chambers) and allow mixing of the two components when desired by a user prior to dispensing. Alternatively or in addition, the two chambers may be configured to allow mixing as the components are being dispensed into the human or animal body.

The drug delivery devices and drugs described herein can be used for the treatment and/or prophylaxis of many different types of disorders. Exemplary disorders include, e.g., diabetes mellitus or complications associated with diabetes mellitus such as diabetic retinopathy, thromboembolism disorders such as deep vein or pulmonary thromboembolism. Further exemplary disorders are acute coronary syndrome (ACS), angina, myocardial infarction, cancer, macular degeneration, inflammation, hay fever, atherosclerosis and/or rheumatoid arthritis.

Exemplary drugs for the treatment and/or prophylaxis of diabetes mellitus or complications associated with diabetes mellitus include an insulin, e.g., human insulin, or a human insulin analogue or derivative, a glucagon-like peptide (GLP-1), GLP-1 analogues or GLP-1 receptor agonists, or an analogue or derivative thereof, a dipeptidyl peptidase-4 (DPP4) inhibitor, or a pharmaceutically acceptable salt or solvate thereof, or any mixture thereof. As used herein, the term "derivative" refers to any substance which is sufficiently structurally similar to the original substance so as to have substantially similar functionality or activity (e.g., therapeutic effectiveness).

Exemplary insulin analogues are Gly(A21), Arg(B31), Arg(B32) human insulin (insulin glargine); Lys(B3), Glu(B29) human insulin; Lys(B28), Pro(B29) human insulin; Asp(B28) human insulin; human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Exemplary insulin derivatives are, for example, B29-N-myristoyl-des(B30) human insulin; B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-gamma-glutamyl)-des(B30) human insulin; B29-N-(N-lithocholyl-gamma-glutamyl)-des(B30) human insulin; B29-N-(ω-carboxyheptadecanoyl)-des(B30) human insulin and B29-N-(ω-carboxyhepta¬decanoyl) human insulin. Exemplary GLP-1, GLP-1 analogues and GLP-1 receptor agonists are, for example: Lixisenatide / AVE0010 / ZP10 / Lyxumia, Exenatide / Exendin-4 / Byetta / Bydureon / ITCA 650 / AC-2993 (a 39 amino acid peptide which is produced by the salivary glands of the Gila monster), Liraglutide / Victoza, Semaglutide, Taspoglutide, Syncria / Albiglutide, Dulaglutide, rExendin-4, CJC-1134-PC, PB-1023, TTP-054, Langlenatide / HM-11260C, CM-3, GLP-1 Eligen, ORMD-0901, NN-9924, NN-9926, NN-9927, Nodexen, Viador-GLP-1, CVX-096, ZYOG-1, ZYD-1, GSK-2374697, DA-3091, MAR-701, MAR709, ZP-2929, ZP-3022, TT-401, BHM-034. MOD-6030, CAM-2036, DA-15864, ARI-2651, ARI-2255, Exenatide-XTEN and Glucagon-Xten.

An exemplary oligonucleotide is, for example: mipomersen / Kynamro, a cholesterol-reducing antisense therapeutic for the treatment of familial hypercholesterolemia.

Exemplary DPP4 inhibitors are Vildagliptin, Sitagliptin, Denagliptin, Saxagliptin, Berberine.

Exemplary hormones include hypophysis hormones or hypothalamus hormones or regulatory active peptides and their antagonists, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, and Goserelin.

Exemplary polysaccharides include a glucosaminoglycane, a hyaluronic acid, a heparin, a low molecular weight heparin or an ultra-low molecular weight heparin or a derivative thereof, or a sulphated polysaccharide, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium. An example of a hyaluronic acid derivative is Hylan G-F 20 / Synvisc, a sodium hyaluronate.

The term "antibody", as used herein, refers to an immunoglobulin molecule or an antigen-binding portion thereof. Examples of antigen-binding portions of immunoglobulin molecules include F(ab) and F(ab')2 fragments, which retain the ability to bind antigen. The antibody can be polyclonal, monoclonal, recombinant, chimeric, de-immunized or humanized, fully human, non-human, (e.g., murine), or single chain antibody. In some embodiments, the antibody has effector function and can fix complement. In some embodiments, the antibody has reduced or no ability to bind an Fc receptor. For example, the antibody can be an isotype or subtype, an antibody fragment or mutant, which does not support binding to an Fc receptor, e.g., it has a mutagenized or deleted Fc receptor binding region.

The terms "fragment" or "antibody fragment" refer to a polypeptide derived from an antibody polypeptide molecule (e.g., an antibody heavy and/or light chain polypeptide) that does not comprise a full-length antibody polypeptide, but that still comprises at least a portion of a full-length antibody polypeptide that is capable of binding to an antigen. Antibody fragments can comprise a cleaved portion of a full length antibody polypeptide, although the term is not limited to such cleaved fragments. Antibody fragments that are useful in the present invention include, for example, Fab fragments, F(ab')2 fragments, scFv (single-chain Fv) fragments, linear antibodies, monospecific or multispecific antibody fragments such as bispecific, trispecific, and multispecific antibodies (e.g., diabodies, triabodies, tetrabodies), minibodies, chelating recombinant antibodies, tribodies or bibodies, intrabodies, nanobodies, small modular immunopharmaceuticals (SMIP), binding-domain immunoglobulin fusion proteins, camelized antibodies, and VHH containing antibodies. Additional examples of antigen-binding antibody fragments are known in the art.

The terms "Complementarity-determining region" or "CDR" refer to short polypeptide sequences within the variable region of both heavy and light chain polypeptides that are primarily responsible for mediating specific antigen recognition. The term "framework region" refers to amino acid sequences within the variable region of both heavy and light chain polypeptides that are not CDR sequences, and are primarily responsible for maintaining correct positioning of the CDR sequences to permit antigen binding. Although the framework regions themselves typically do not directly participate in antigen binding, as is known in the art, certain residues within the framework regions of certain antibodies can directly participate in antigen binding or can affect the ability of one or more amino acids in CDRs to interact with antigen.

Exemplary antibodies are anti PCSK-9 mAb (e.g., Alirocumab), anti IL-6 mAb (e.g., Sarilumab), and anti IL-4 mAb (e.g., Dupilumab).

The compounds described herein may be used in pharmaceutical formulations comprising (a) the compound(s) or pharmaceutically acceptable salts thereof, and (b) a pharmaceutically acceptable carrier. The compounds may also be used in pharmaceutical formulations that include one or more other active pharmaceutical ingredients or in pharmaceutical formulations in which the present compound or a pharmaceutically acceptable salt thereof is the only active ingredient. Accordingly, the pharmaceutical formulations of the present disclosure encompass any formulation made by admixing a compound described herein and a pharmaceutically acceptable carrier.

Pharmaceutically acceptable salts of any drug described herein are also contemplated for use in drug delivery devices. Pharmaceutically acceptable salts are for example acid addition salts and basic salts. Acid addition salts are e.g. HCl or HBr salts. Basic salts are e.g. salts having a cation selected from an alkali or alkaline earth metal, e.g. Na+, or K+, or Ca2+, or an ammonium ion N+(R1)(R2)(R3)(R4), wherein R1 to R4 independently of each other mean: hydrogen, an optionally substituted C1 C6-alkyl group, an optionally substituted C2-C6-alkenyl group, an optionally substituted C6-C10-aryl group, or an optionally substituted C6-C10-heteroaryl group. Further examples of pharmaceutically acceptable salts are known to those of skill in the arts. Pharmaceutically acceptable solvates are for example hydrates or alkanolates such as methanolates or ethanolates.

Those of skill in the art will understand that modifications (additions and/or removals) of various components of the substances, formulations, apparatuses, methods, systems and embodiments described herein may be made without departing from the full scope of the present invention, which encompass such modifications.

## Claims

1. An injection device comprising:
a needle;
a needle sleeve surrounding the needle, the needle sleeve having an aperture and being arranged such that the needle extends through the aperture during an injection process; and
a needle obscuring element reversibly movable between a first position and a second position relative to the needle sleeve, wherein the needle obscuring element is coupled to the needle sleeve when in the first position and when in the second position, and wherein the needle obscuring element is arranged to cover a larger portion of the aperture when in the second position than when in the first position,
wherein the needle obscuring element is biased towards the second position,
wherein the needle obscuring element comprises a plurality of flaps arranged within the needle sleeve, wherein the plurality of flaps are pivotally coupled to the needle sleeve such that the plurality of flaps can pivot between the first position and the second position around axes of rotation substantially parallel to the aperture,
wherein the needle obscuring element is arranged to move from the first position to the second position in response to removal of a cap from the needle sleeve of the injection device, and
wherein the needle obscuring element is arranged to move from the second position to the first position in response to insertion of at least part of the cap into the needle sleeve of the injection device.

2. An injection device according to claim 1, wherein the injection device further comprises a housing, wherein the needle is fixed relative to the housing, and wherein the needle sleeve is configured to move into the housing to expose the needle.

3. An injection device according to claim 1, wherein the injection device further comprises a housing, wherein the needle sleeve is fixed relative to the housing, and wherein the needle is configured to move relative to the needle sleeve to expose the needle.

4. An injection device according to any preceding claim, wherein the at least one flap is coupled to the needle sleeve by a living hinge, wherein the at least one flap is biased towards the second position by the living hinge.

5. An injection device according to any preceding claim, wherein the needle obscuring element is arranged to have an opening when in the second position, through which the needle may extend during the injection process.

6. An injection device according to any preceding claim, wherein the needle obscuring element is integrally formed with the needle sleeve.

7. An injection device according to any preceding claim, wherein the needle obscuring element is configured to lock in the second position in response to moving from the first position to the second position.

8. An injection device according to any preceding claim, further comprising the cap, the cap having a needle shield,
wherein the needle shield is at least partially located within the needle sleeve, and
wherein the needle obscuring element is held in the first position by the needle shield when the needle shield is at least partially located within the needle sleeve.

9. An injection device according to any preceding claim, wherein the injection device is an auto-injector, a pen-injector, a safety syringe, or a large-volume injection device.

10. An injection device according to any preceding claim, further comprising a container containing a medicament.

## Patentansprüche

1. Injektionsvorrichtung, umfassend:
eine Nadel,
eine die Nadel umgebende Nadelhülse, wobei die Nadelhülse eine Öffnung aufweist und so angeordnet ist, dass sich die Nadel während eines Injektionsvorgangs durch die Öffnung erstreckt, und
ein Nadelverdeckelement, das reversibel zwischen einer ersten Position und einer zweiten Position relativ zu der Nadelhülse beweglich ist, wobei das Nadelverdeckelement an die Nadelhülse gekoppelt ist, wenn es sich in der ersten Position befindet und wenn es sich in der zweiten Position befindet, und wobei das Nadelverdeckelement so angeordnet ist, dass es in der zweiten Position einen größeren Abschnitt der Öffnung abdeckt, als wenn es sich in der ersten Position befindet,
wobei das Nadelverdeckelement auf die zweite Position hin vorgespannt ist,
wobei das Nadelverdeckelement eine Vielzahl von Laschen umfasst, die in der Nadelhülse angeordnet sind, wobei die Vielzahl von Laschen schwenkbar an die Nadelhülse gekoppelt sind, so dass die Vielzahl von Laschen zwischen der ersten Position und der zweiten Position um Drehachsen, die im Wesentlichen parallel zu der Öffnung verlaufen, schwenken können,
wobei das Nadelverdeckelement so angeordnet ist, dass es sich als Reaktion auf das Entfernen einer Kappe von der Nadelhülse der Injektionsvorrichtung aus der ersten Position in die zweite Position bewegt, und
wobei das Nadelverdeckelement so angeordnet ist, dass es sich als Reaktion auf das Einführen mindestens eines Teils der Kappe in die Nadelhülse der Injektionsvorrichtung aus der zweiten Position in die erste Position bewegt.

2. Injektionsvorrichtung nach Anspruch **1,** wobei die Injektionsvorrichtung ferner ein Gehäuse umfasst, wobei die Nadel relativ zu dem Gehäuse fixiert ist und wobei die Nadelhülse dazu ausgestaltet ist, sich in das Gehäuse zu bewegen, um die Nadel freizulegen.

3. Injektionsvorrichtung nach Anspruch 1, wobei die Injektionsvorrichtung ferner ein Gehäuse umfasst, wobei die Nadelhülse relativ zu dem Gehäuse fixiert ist und wobei die Nadel dazu ausgestaltet ist, sich relativ zu der Nadelhülse zu bewegen, um die Nadel freizulegen.

4. Injektionsvorrichtung nach einem der vorhergehenden Ansprüche, wobei die mindestens eine Lasche durch ein Filmscharnier an die Nadelhülse gekoppelt ist, wobei die mindestens eine Lasche durch das Filmscharnier zu der zweiten Position hin vorgespannt ist.

5. Injektionsvorrichtung nach einem der vorhergehenden Ansprüche, wobei das Nadelverdeckelement so angeordnet ist, dass es, wenn es sich in der zweiten Position befindet, eine Öffnung aufweist, durch die sich die Nadel während des Injektionsvorgangs erstrecken kann.

6. Injektionsvorrichtung nach einem der vorhergehenden Ansprüche, wobei das Nadelverdeckelement integral mit der Nadelhülse ausgebildet ist.

7. Injektionsvorrichtung nach einem der vorhergehenden Ansprüche, wobei das Nadelverdeckelement dazu ausgestaltet ist, sich als Reaktion auf das Bewegen aus der ersten Position in die zweite Position in der zweiten Position zu verriegeln.

8. Injektionsvorrichtung nach einem der vorhergehenden Ansprüche, ferner umfassend die Kappe, wobei die Kappe einen Nadelschutz aufweist,
wobei sich der Nadelschutz mindestens teilweise in der Nadelhülse befindet und
wobei das Nadelverdeckelement durch den Nadelschutz in der ersten Position gehalten wird, wenn sich der Nadelschutz mindestens teilweise in der Nadelhülse befindet.

9. Injektionsvorrichtung nach einem der vorhergehenden Ansprüche, wobei die Injektionsvorrichtung ein Autoinjektor, ein Pen-Injektor, eine Sicherheitsspritze oder eine großvolumige Injektionsvorrichtung ist.

10. Injektionsvorrichtung nach einem der vorhergehenden Ansprüche, ferner umfassend einen Behälter, der ein Medikament enthält.

## Revendications

1. Dispositif d'injection comprenant :
une aiguille ;
un manchon d'aiguille entourant l'aiguille, le manchon d'aiguille ayant une ouverture et étant disposé de telle sorte que l'aiguille s'étend à travers l'ouverture pendant un processus d'injection ; et
un élément masquant l'aiguille pouvant être déplacé de manière réversible entre une première position et une seconde position par rapport au manchon d'aiguille, dans lequel l'élément masquant l'aiguille est accouplé au manchon d'aiguille lorsqu'il est dans la première position et lorsqu'il est dans la seconde position, et dans lequel l'élément masquant l'aiguille est conçu pour couvrir une plus grande partie de l'ouverture lorsqu'il est dans la seconde position que lorsqu'il est dans la première position,
dans lequel l'élément masquant l'aiguille est sollicité vers la seconde position,
dans lequel l'élément masquant l'aiguille comprend une pluralité de volets disposés à l'intérieur du manchon d'aiguille, dans lequel la pluralité de volets est accouplée de manière pivotante au manchon d'aiguille de telle sorte que la pluralité de volets peut pivoter entre la première position et la seconde position autour d'axes de rotation sensiblement parallèles à l'ouverture,
dans lequel l'élément masquant l'aiguille est conçu pour se déplacer de la première position à la seconde position en réponse au retrait d'un capuchon depuis le manchon d'aiguille du dispositif d'injection, et
dans lequel l'élément masquant l'aiguille est conçu pour se déplacer de la seconde position à la première position en réponse à l'insertion d'au moins une partie du capuchon dans le manchon d'aiguille du dispositif d'injection.

2. Dispositif d'injection selon la revendication 1, dans lequel le dispositif d'injection comprend en outre un logement, dans lequel l'aiguille est fixe par rapport au logement, et dans lequel le manchon d'aiguille est configuré pour se déplacer dans le logement pour faire apparaître l'aiguille.

3. Dispositif d'injection selon la revendication 1, dans lequel le dispositif d'injection comprend en outre un logement, dans lequel le manchon d'aiguille est fixe par rapport au logement, et dans lequel l'aiguille est configurée pour se déplacer par rapport au manchon d'aiguille pour faire apparaître l'aiguille.

4. Dispositif d'injection selon une quelconque revendication précédente, dans lequel l'au moins un volet est accouplé au manchon d'aiguille par une charnière souple, dans lequel l'au moins un volet est sollicité vers la seconde position par la charnière souple.

5. Dispositif d'injection selon une quelconque revendication précédente, dans lequel l'élément masquant l'aiguille est conçu pour avoir une ouverture lorsqu'il est dans la seconde position, à travers laquelle l'aiguille peut s'étendre pendant le processus d'injection.

6. Dispositif d'injection selon une quelconque revendication précédente, dans lequel l'élément masquant l'aiguille est formé d'un seul tenant avec le manchon d'aiguille.

7. Dispositif d'injection selon une quelconque revendication précédente, dans lequel l'élément masquant l'aiguille est configuré pour se bloquer dans la seconde position en réponse au déplacement de la première position à la seconde position.

8. Dispositif d'injection selon une quelconque revendication précédente, comprenant en outre le capuchon, le capuchon ayant un protège-aiguille,
dans lequel le protège-aiguille est au moins partiellement situé à l'intérieur du manchon d'aiguille, et
dans lequel l'élément masquant l'aiguille est maintenu dans la première position par le protège-aiguille lorsque le protège-aiguille est au moins partiellement situé à l'intérieur du manchon d'aiguille.

9. Dispositif d'injection selon une quelconque revendication précédente, dans lequel le dispositif d'injection est un auto-injecteur, un stylo-injecteur, une seringue de sécurité ou un dispositif d'injection de grand volume.

10. Dispositif d'injection selon une quelconque revendication précédente, comprenant en outre un récipient contenant un médicament.
